# EUROPEAN PATENT APPLICATION

(11) **EP 3 770 268 A1**
(43) Date of publication of application: **27.01.2021**
(21) Application number: 19772153.3
(22) Date of filing: 19.03.2019
(51) Int. Cl.: C12P 19/30, C12N 9/12, C12P 19/32

(54) **METHOD FOR PRODUCING ß-NMN AND COMPOSITION CONTAINING SAME**

(30) Priority: 20.03.2018 JP 2018053238
(71) Applicant: Mitsubishi Corporation Life Sciences Limited, Tokyo 100-0006 (JP)
(72) Inventor: FUKAMIZU, Yuichiro, Saiki-shi Oita 876-8580 (JP)
(74) Representative: Müller-Boré & Partner Patentanwälte PartG mbB
(86) International application number: PCT/JP2019/011527
(87) International publication number: WO 2019/181961

(57) **Abstract**

[Problem] To obtain β-NMN from a yeast having been used in foods, and to obtain a β-NMN composition from a composition containing NAD. [Solution] The present inventor found out that β-NMN is produced by reacting a metabolic composition, said metabolic composition being prepared by adding a crude enzyme prepared from a microorganism belonging to the genus *Aspergillus* to, for example, a composition containing NAD such as a yeast extract, under specific conditions. Namely, β-NMN is produced by the crude enzyme, etc. prepared from a microorganism belonging to the genus *Aspergillus* with the use of NAD that is contained in the yeast extract as a substrate.

## Description

### Technical Field

The present invention relates to a method for producing β-nicotinamide mononucleotide (β-NMN), the method including allowing a metabolic composition, a crude enzyme or a purified enzyme prepared from the genus Aspergillus such as Aspergillus oryzae, to react with β-nicotinamide adenine dinucleotide (NAD) or an NAD-containing solution obtained for example from yeast Candida utilis; and a composition that contains β-NMN.

### Background Art

β-Nicotinamide mononucleotide (β-NMN) is an intermediate metabolite of β-nicotinamide adenine dinucleotide (NAD) in in vivo salvage pathway (Patent Literatures 1 to 4, Non Patent Literature 1). Upon being introduced in vivo, β-NMN can directly induce biosynthesis of NAD, and can thereby elevate in vivo NAD concentration (Non Patent Literature 2). By use of β-NMN, while employing substrate NAD whose biosynthesis has been induced, sirtuin family proteins or the like represented by SIRT1 which are NAD-dependent deacetylases (SIRT2, SIRT3, SIRT4, SIRT5, SIRT6, SIRT7) are activated. With the sirtuin family proteins or the like activated, expressed now are a wide variety of lifetime-related physiological activities including metabolic improvement, anti-disease function and anti-aging function (Non Patent Literature 1). Reported functions pertained by β-NMN are detailed as "improvement of saccharometabolism abnormality (Non Patent Literature 2)", "involvement to circadian rhythm (Non Patent Literatures 3) 4)", "functional improvement of aged mitochondria (Non Patent Literature 5)", "protection of heart from ischemia and reperfusion (Non Patent Literature 6)", "suppression of aging-related decrease of neural stem cell (Non Patent Literature 7)", "suppression of Claudin-1 expression by epigenetic control mechanism, and concomitant reduction of albuminuria in diabetic nephropathy (Non Patent Literature 8)", "regulation of programmed cell death (Non Patent Literature 9)", "improvement of Parkinson's disease (Non Patent Literature 10)", and "recovery from aging-related oxidative stress and vascular dysfunction (Non Patent Literature 11)". β-NMN has been also known not only to activate the sirtuin family proteins or the like, but also to improve eye functions (Patent Literature 5). As described above, many of negative physiological events in cells, tissues and organs are expected to be cured, improved or prevented, by administering β-NMN so as to enhance biosynthesis of NAD and to activate SIRT1 and other sirtuin family proteins including SIRT2, SIRT3, SIRT4, SIRT5, SIRT6 and SIRT7.

Yeasts have been used for various foods and so forth. In particular, torula yeast (Candida utilis) is an edible yeast verified to be safe by the Food and Drug Administration (FDA) on the basis of its high trophicity and history of safe use. Thereby, torula yeast has been efficiently used for drug, supplement, seasoning and so forth over the years.

### Citation List

### Patent Literature

Patent Literature 1: WO 2014/146044 A
Patent Literature 2: CN 101601679 B
Patent Literature 3: US 2011-0123510 A1
Patent Literature 4: US 7737158
Patent Literature 5: WO 2016/171152 A

### Non Patent Literature

Non Patent Literature 1: Liana, R Stein.et al. The dynamic regulation of NAD metabolism in mitochondria. Trends in Endocrinology and Metabolism. 2012, Vol. 23, No. 9
Non Patent Literature 2: J, Yoshino.et al. Nicotinamide Mononucleotide, a Key NAD+ Intermediate, Treats the Pathophysiology of Diet- and Age-Induced Diabetes in Mice. Cell Metab. 2011, 14(4), P.528-536.
Non Patent Literature 3: Clara Bien Peek1.et al. Circadian Clock NAD+ Cycle Drives Mitochondrial Oxidative Metabolism in Mice. Science. 2013, 342(6158), 1243417.
Non Patent Literature 4: Ramsey, KM.et al. Circadian clock feedback cycle through NAMPT-mediated NAD+ biosynthesis. Science. 2009, 324(5927), P.651-654.
Non Patent Literature 5: Ana, P.Gomes.et al. Declining NAD+ Induces a Pseudohypoxic State Disrupting Nuclear-Mitochondrial Communication during Aging. Cell. 2013, 155(7), P.1624-1638.
Non Patent Literature 6: T, Yamamoto.et al. Nicotinamide mononucleotide, an intermediate of NAD+ synthesis, protects the heart from ischemia and reperfusion. PLoS One. 2014, 9(6), e98972.
Non Patent Literature 7: Liana, R Stein.et al. Specific ablation of Nampt in adult neural stem cells recapitulates their functional defects during aging. EMBO J. 2014, 33(12), P.1321-1340
Non Patent Literature 8: K, Hasegawa.et al. Renal tubular Sirtl attenuates diabetic albuminuria by epigenetically suppressing Claudin-1 overexpression in podocytes. Nat Med. 2013, 19(11), P.1496-1504
Non Patent Literature 9: Nicolas Preyat.et al. Complex role of nicotinamide adenine dinucleotide in the regulation of programmed cell death pathways. Biochem Pharmacology. 2015, S0006-2952(15)
Non Patent Literature 10: Lei lu.et al. Nicotinamide mononucleotide improves energy activity and survival rate in an in vitro model of Parkinson's disease. Exp Ther Med. 2014, 8(3), P.943-950.
Non Patent Literature 11: Natalie E.de Picciotto.et al. Nicotinamide mononucleotide supplementation reverses vascular dysfunction and oxidative stress with aging in mice. Aging cell. 2016, 15, P.522-530.

### Summary of Invention

### Technical Problem

It is an object of the present invention to obtain an enzyme capable of producing β-NMN, a method of producing β-NMN also applicable to food, and for example to obtain a β-NMN-enriched composition from a yeast with history of safe use.

### Solution to Problem

The present inventor found that β-NMN is efficiently obtainable by an optimized enzyme reaction (at 50 to 70°C and pH3.0 to 7.0), by using a metabolic composition, a crude enzyme, or a purified enzyme obtained from a microorganism that belongs to the genus Aspergillus, and arrived at the present invention.

The present invention is an invention as following.
(A) A method for producing β-nicotinamide mononucleotide, the method comprising performing a reaction of β-nicotinamide adenine dinucleotide which is a substrate, using a metabolic composition of a microorganism that belongs to the genus Aspergillus.
(B) A method for producing β-nicotinamide mononucleotide, the method comprising performing a reaction using an enzymatic protein derived from a microorganism that belongs to the genus Aspergillus and having properties listed below:
   (1) action: hydrolyzes a phosphoanhydride bond (pyrophosphate bond), which is a high-energy phosphate bond in a β-nicotinamide adenine dinucleotide structure, to produce β-nicotinamide mononucleotide and AMP;
   (2) pyrophosphatase activity;
   (3) optimum pH: pH3.0 to 7.0; and
   (4) optimum temperature: 40°C to 70°C.
(C) A method for producing a composition that contains 1% (w/w) or more of β-nicotinamide mononucleotide, the method comprising performing a reaction of β-nicotinamide adenine dinucleotide contained in an extract of yeast, or β-nicotinamide adenine dinucleotide which is a substrate, using an enzyme derived from a microorganism that belongs to the genus Aspergillus and having properties listed below:
   (1) action: hydrolyzes a phosphoanhydride bond (pyrophosphate bond), which is a high-energy phosphate bond in a β-nicotinamide adenine dinucleotide structure, to produce β-nicotinamide mononucleotide;
   (2) pyrophosphatase activity;
   (3) optimum pH: pH3.0 to 7.0; and
   (4) optimum temperature: 40°C to 70°C.
(D) An enzyme derived from a microorganism that belongs to the genus Aspergillus, having physicochemical properties listed below:
   (1) action: hydrolyzes a phosphoanhydride bond (pyrophosphate bond), which is a high-energy phosphate bond in a β-nicotinamide adenine dinucleotide structure, to produce β-nicotinamide mononucleotide;
   (2) pyrophosphatase activity;
   (3) optimum pH: pH3.0 to 7.0; and
   (4) optimum temperature: 40°C to 70°C.

### Advantageous Effects of Invention

According to the present invention, β-NMN is obtainable by using NAD as a substrate. For example, β-NMN is easily obtainable for example from a yeast extract with history of safe use. In particular, torula yeast is a yeast with a long-known history of safe use, and a yeast extract obtained therefrom is proven to be highly safe. Such β-NMN-rich yeast extract is ingestible as drug, supplement, functional food and so forth. Since the β-NMN-rich yeast extract of the present invention can also produce AMP (5'-adenylic acid), so that the yeast extract is employable not only as a β-NMN-rich yeast extract, but also as an AMP-rich yeast extract.

### Brief Description of Drawings

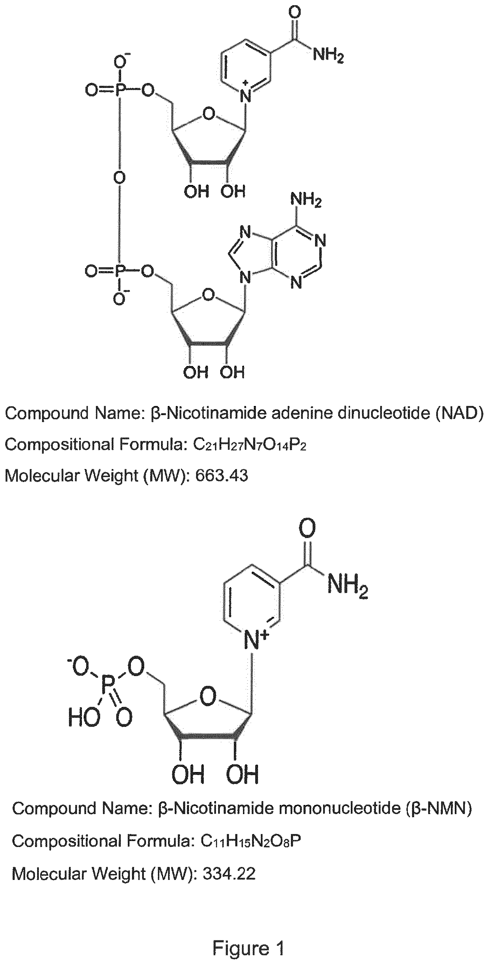

Fig. 1 illustrates molecular structures, compositional formulae and molecular weights of the β-NMN and NAD.
Fig. 2 illustrates a reaction model of a composition that contains β-NMN obtainable by a reaction of a purified enzyme or a crude enzyme obtained from a microorganism that belongs to the genus Aspergillus, with an extraction liquid that contains NAD as a substrate.
Fig. 3 illustrates an optimum reaction condition 1 (30°C/pH1, 2, 3, 4, 5, 6, 7, 8, 9, 10).
Fig. 4 illustrates an optimum reaction condition 2 (40°C/pH1, 2, 3, 4, 5, 6, 7, 8, 9, 10).
Fig. 5 illustrates an optimum reaction condition 3 (50°C/pH1, 2, 3, 4, 5, 6, 7, 8, 9, 10).
Fig. 6 illustrates an optimum reaction condition 4 (60°C/pH1, 2, 3, 4, 5, 6, 7, 8, 9, 10).
Fig. 7 illustrates an optimum reaction condition 5 (70°C/pH1, 2, 3, 4, 5, 6, 7, 8, 9, 10).
Fig. 8 illustrates an optimum reaction condition 6 (80°C/pH1, 2, 3, 4, 5, 6, 7, 8, 9, 10).

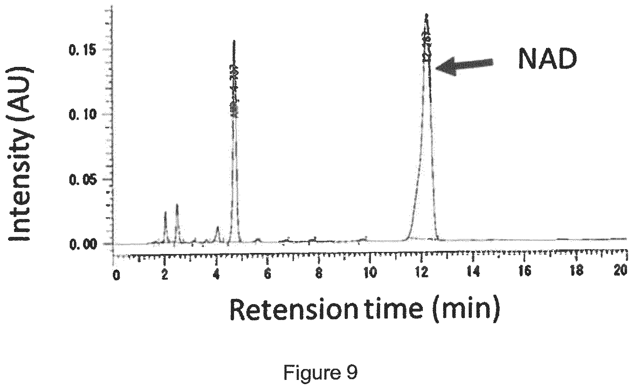

Fig. 9 is a chromatogram that indicates NAD in a yeast extract obtained by a reaction between an extraction liquid prepared from Candida utilis IAM 4264 and a crude enzyme derived from Aspergillus niger.
Fig. 10 is a chromatogram that indicates a β-NMN-containing composition in a yeast extract obtained by a reaction between an extraction liquid prepared from Candida utilis IAM 4264 and a crude enzyme derived from Aspergillus niger.

### Description of Embodiments

A metabolic composition, a crude enzyme, or a purified enzyme used in the present invention is an enzyme capable of producing β-NMN from NAD as a substrate, wherein NAD used as a substrate may be contained in a yeast or in a yeast extract. An NAD source that is any of those normally available may be used in the composition of the present invention.

In a case where the yeast extract is employed, edible yeasts may be used as the yeast, which are exemplified by those belong to the genus Saccharomyces, the genus Kluyveromyces, the genus Candida, and the genus Pichia. Among them, preferred is Candida utilis that belongs to the genus Candida. More specific examples thereof include Candida utilis IAM 4264, Candida utilis ATCC 9950, Candida utilis ATCC 9550, Candida utilis IAM 4233, and Candida utilis AHU 3259. Furthermore preferably, use of a glutathione-rich yeast increases β-NMN content. The glutathione-rich yeast employable here may be any of those obtainable by known methods (see, e.g., JP S59-151894 A and JP S60-156379 A).

Culture medium for culturing the yeast uses a carbon source such as glucose, acetic acid, ethanol, glycerol, molasses, spent sulfite liquid or the like; and uses a nitrogen source such as urea, ammonia, ammonium sulfate, ammonium chloride, and nitrate or the like. Also phosphate, potassium and magnesium sources may be any of ordinary industrial materials such as calcium superphosphate, ammonium phosphate, potassium chloride, potassium hydroxide, magnesium sulfate and magnesium chloride, and still other inorganic salt containing zinc, copper, manganese, iron or other ion are added. Although the culture is sustainable without using other ingredients such as vitamin, amino acid and nucleic acid-related substance, they may otherwise be added. Also an organic substance such as corn steep liquor, casein, yeast extract, meat extract or peptone may be added.

Any culture conditions such as culture temperature, pH and so forth are applicable without special limitation, and may only be determined according to yeast strain to be used, and then cultured. In many cases, the culture temperature is 21 to 37°C, preferably 25 to 34°C, meanwhile pH is 3.0 to 8.0, and particularly preferably 3.5 to 7.0.

Culture format, which may either be batch culture or continuous culture, is preferably the latter from an industrial viewpoint. Stirring, aeration and so forth during culture may employ conditions not specifically limited, and may follow any of known methods.

Cultured cells are then pre-treated for preparation of an extraction liquid. Wet yeast cells after the cell culture are washed by repeating suspension in deionized water and centrifugation, and then subjected to extraction. Method for the extraction may suitably be controlled depending on species of the yeast cells to be used. In view of increasing the β-NMN content, the method is preferably conducted under conditions where NAD and β-NMN in the yeast will not be decomposed. The method is any of self-digestion, alkali extraction, acid extraction, hot water extraction, or combination of them. In an exemplary case with Candida utilis, the cells are re-suspended in deionized water so as to adjust the cell concentration to 7 to 10% on the dry weight basis, preferably to 8 to 9%. In the process of extraction from the cell suspension, pH is adjusted as necessary. In an exemplary case of acid extraction, pH during extraction is adjusted to 1.5 to 7.0, most preferably at around 6.0. The pH adjustment may follow any of known methods.

Extraction temperature is set to 50 to 90°C, preferably 50 to 65°C. Any of known methods is employable for the temperature adjustment without special limitation, so long as the extraction liquid can be adjusted to the aforementioned temperature.

Extraction time may only be 5 minutes or longer. The extraction is preferably accompanied by stirring. Stirring speed and so forth may be suitably adjusted without special limitation. An extraction time of 40 to 50 minutes is further preferred, since the β-NMN content will increase.

After the extraction, the cell suspension is centrifuged to remove the cells, and to obtain a supernatant. The supernatant is defined to be an extraction liquid, and is used as a substrate solution for the enzyme reaction in the present invention. Although the liquid extracted from the yeast according to the aforementioned method may be used intact in the method of the present application, an alternative method may be such as, for example, purifying and condensing NAD in the yeast extraction liquid and then going into the succeeding steps.

The enzyme used here is an enzyme capable of producing β-NMN from a substrate, which is NAD contained in the solution having been obtained in the preceding steps. More specifically, enzymes derived from microorganisms that belong to the genus Aspergillus are used. The genus Aspergillus is exemplified by Aspergillus melleus (NBRC 4339, etc.), Aspergillus oryzae (NBRC 100959, etc.), and Aspergillus niger (ATCC 10254, etc.), among which employable is an enzyme derived from the genus Aspergillus with history of safe use.

The enzyme employed in the present invention may be a crude enzyme prepared from microorganisms in the genus Aspergillus as described above. The microorganisms in the genus Aspergillus may be of any strain having been used in food industry and so forth. The microorganisms in the genus Aspergillus may be any of those normally available. The microorganisms in the genus Aspergillus may be any strain available from strain distribution organizations such as ATCC and NBRC, or commercially available from seed/strain sales company. The crude enzyme used in the present invention may be prepared by any of well-known methods. Medium may be any of well-known media used for the genus Aspergillus, and also a protein composition after culture may be prepared by any of well-known methods. A fraction containing a protein group such as enzyme may be obtained, for example, by methods for culturing strain, and rough purification process that involves extraction or solvent fractionation, as described in JP 2010-004760 A and JP 2009-232835 A.

More specifically, spore of a microorganism in the genus Aspergillus, such as Aspergillus. oryzae No. 2007 (from Higuchi Matsunosuke Shoten Co., Ltd.), is cultured in a liquid microorganism culture medium (potato dextrose medium). Culture conditions normally employed include a pH of 5.5 to 8.5, preferably a pH of 6 to 8, and a temperature of 25 to 42°C, preferably 30 to 37°C. Culture time is normally around 2 to 7 days, which may vary depending on strain to be used and other conditions.

The present application can employ a metabolic composition that contains a crude enzyme obtained from the microorganism in the genus Aspergillus, a crude enzyme or a purified enzyme. The metabolic composition that contains a crude enzyme is a fraction containing a protein group fractionated from the culture supernatant, or, a fraction obtained by disrupting the microorganism such as Aspergillus niger in the culture liquid, and by collecting a fraction that contains the intracellular protein group. When using the crude enzyme or the purified enzyme, employable is a purpose-specific crude enzyme or a purified enzyme isolated and collected by any of known protein purification methods, such as various chromatographic processes including ion exchange chromatography, normal phase chromatography, reverse phase chromatography, gel filtration chromatography, gel permeation chromatography, absorption chromatography, hydrophilic interaction chromatography, affinity chromatography, and supercritical chromatography. The enzyme may be a dry product obtained after drying process. Enzymes derived from the genus Aspergillus have been marketed in a wide variety, and many of which contains contaminant enzymes, so that also the enzyme applicable to the method of the present application is available.

The above-described enzyme was firstly discovered by the present inventor to have an enzymatic activity capable of producing β-NMN from NAD as a substrate, and is employable as an enzyme capable of producing β-NMN from a substrate which is not only NAD in the yeast, but also an NAD-containing composition other than yeast, or pure NAD. NAD may be any of normally available ones.

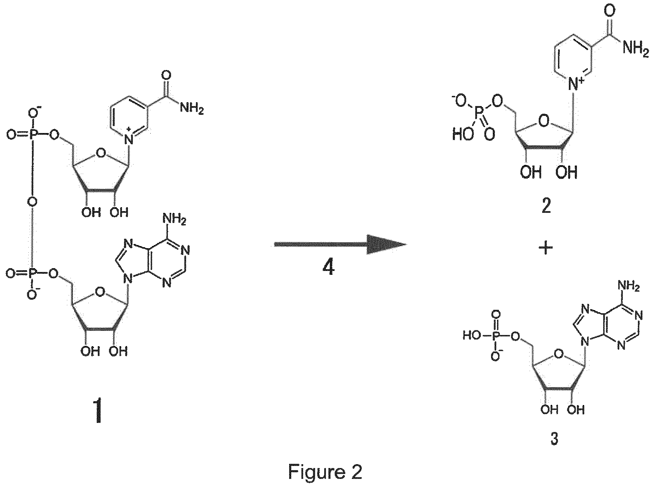

The enzyme used in the present invention has a pyrophosphatase activity that cleaves a high-energy phosphate bond, such as a pyrophosphate bond (phosphoanhydride bond) of NAD. Reaction of the present invention is illustrated in Fig. 2. The enzyme used in the present invention, having such activity, produces a composition that contains β-NMN and adenosine 5'-monophosphate (AMP, 5'-adenylic acid), as a product from the substrate NAD.

With the aforementioned activity of the enzyme used in the present invention, a culture that involves the substrate NAD in the yeast extract can yield a yeast extract that contains β-NMN and AMP. When using NAD in the yeast extract as a substrate, there may be a process of enriching NAD in the yeast extract. For example, after extracting the yeast extract, the extract may be allowed to pass through a cation exchange resin or the like, commonly used in food manufacturing, to condense NAD in the yeast extract. Content of β-NMN in the yeast extract varies depending on the NAD content before the enzymatic reaction, wherein the larger the NAD content, the larger the β-NMN content after the reaction.

Amount of addition of the enzyme used for the reaction varies depending on methods for preparing the enzyme, wherein the enzyme is normally added so as to adjust the total protein content to 0.1% (w/w) to 20 (w/w), relative to the total substrate NAD contained in the solution. The amount of addition is more preferably 1 to 10 (w/w). Note that β-NMN and a β-NMN-containing composition used for investigating optimum reaction conditions of the enzyme in the present application were measured under HPLC conditions described later in Examples.

Optimum temperature of a reaction that uses the crude enzyme is 40 to 70°C, preferably 50°C to 60°C, and more preferably 60°C. Note that β-NMN and a β-NMN-containing composition used for investigating optimum reaction conditions of the enzyme in the present application were measured under HPLC conditions described later in Examples.

Optimum pH of a reaction that uses the crude enzyme is 3.0 to 7.0, preferably 4.0 to 6.0, and more preferably pH 5.0. Note that β-NMN and a β-NMN-containing composition used for investigating optimum reaction conditions of the enzyme in the present application were measured under HPLC conditions described later in Examples.

By adding a whole protein, crude enzyme or purified enzyme derived from the genus Aspergillus to the extraction liquid prepared from the yeast as described above, and by allowing the enzyme reaction to proceed under the optimum reaction conditions, it now becomes possible to obtain, for example, the yeast extract that contains 1.0% (w/w) or more of β-NMN relative to the dry solid matter of the yeast extract. By using a yeast with a large substrate (NAD) content before the enzyme reaction, obtainable is a yeast extract containing 5% (w/w) or more β-NMN, although depending on the degree of condensation of the substrate. Moreover, the yeast extract containing 20% (w/w) or more β-NMN is obtainable, if NAD in the yeast extract is further condensed to 30 to 50% (w/w). Content of β-NMN in the present invention will vary depending on the NAD content in yeast. By increasing the NAD content in the substrate solution resulted after the enzyme reaction for extracting NAD from the yeast cells, it now becomes possible to further enrich the β-NMN content. Note that β-NMN and a β-NMN-containing composition used for investigating optimum reaction conditions of the enzyme in the present application were measured under HPLC conditions described later in Examples.

The extraction liquid that went through the enzyme reaction may further be condensed, followed by lyophilization or hot air drying, to yield a β-NMN-containing yeast extract.

In another mode, by purifying β-NMN from the β-NMN-containing yeast extract, a composition having the yeast derived β-NMN further enriched therein may be obtained. Alternatively, a composition having the yeast derived β-NMN enriched therein may be obtained by purifying β-NMN from the yeast extraction liquid before being dried in the preceding stage. Method for purification may be a well-known chromatographic process by use of activated carbon and ion exchange resin or the like. Note that even in a case where NAD other than that contained in the yeast extract is used as the substrate, β-NMN may be produced in the same way as in the method of using NAD in the yeast extract as the substrate.

Method for ingesting the yeast extract or the yeast-derived β-NMN-containing composition of the present invention is not specifically limited, and is exemplified by oral administration, or non-oral administration such as intravenous, intraperitoneal or subcutaneous administration. More specifically either oral agent such as tablets, powders, granules, pills, suspensions, emulsions, infusions and decoctions, capsules, syrups, liquid, elixir, extract, tinctures and fluidextracts; or non-oral agent such as injections, drops, liniment, plasters, aerosols, buccals, nasal drops, ophthalmic solutions, suppositories, may be used.

The yeast extract produced in the present invention may be ingested not only as drug, but also as food, functional food, dietary supplement, supplement and so forth.

The yeast extract of the present invention may also be used in combination with other ingredient that does not reduce activity of β-NMN, or enhances activity of β-NMN. The ingredient is exemplified by excipient and diluent, such as dextrin, maltitol, sorbitol and starch. The activity of β-NMN is exemplified by sirtuin activity.

Ingestion amount of the present invention may only be an amount with which β-NMN activity can be demonstrated. The amount of administration necessary for demonstration of the β-NMN activity is typically determined, while considering choice of composition to be administered, age, body weight, response and condition of the individual who ingests, and so forth. Normal ingestion is approximately 100 mg/day to 1000 mg/day.

### Examples

The invention of the present application will specifically be explained below, to which the invention of the present application by no means limited.

### <Measurement Conditions of HPLC Used for Detecting β-NMN and Composition>

Pump: Chromaster 5110 (from Hitachi High-Technologies Corporation)
Autosampler: Chromaster 5210 (from Hitachi High-Technologies Corporation)
UV detector: Chromaster 5410 (from Hitachi High-Technologies Corporation)
Column oven: Chromaster 5310 (from Hitachi High-Technologies Corporation)
Mobile phase: 50 mM phosphate buffer (pH2.8)
   · 0.5% ammonium dihydrogen phosphate (from FUJIFILM Wako Pure Chemical Corporation)
   · adjusted to pH2.8 by using 85% phosphoric acid for high performance chromatography (from FUJIFILM Wako Pure Chemical Corporation).
Column: Unison UK-C18 ϕ 4.6 mm × W 150 mm, particle size = 3.0 µm (from Intakt Corporation)
Column oven temperature: 30°C
Flow rate: 1.2 mL/min
Elution mode: isocratic
Detection UV wavelength: 210 nm
Analysis time: 25 minutes
Sample injection volume: 5 µL
Sample cooling temperature: 3°C ± 2°C

### <Preparation of Protein Composition>

Spores of Aspergillus niger (ATCC 10254) in a cut piece of a slant potato dextrose agar medium were cultured in a YPDS liquid medium (starch 30 g, glucose 2.5 g, polypeptone 10 g, yeast extract 5 g, soybean flour 1 g, biotin 0.02 mg, arginine hydrochloride 0.55 g/L) at 30°C for three days. Then 100 µl of the culture supernatant was dried in vacuo to obtain an enzyme composition, which was used to confirm production of β-NMN, as described below.

### <Study on Optimum pH of Enzyme Reaction with Substrate NAD>

Optimum pH of enzyme reaction of the enzyme composition obtained in the previous stage was studied by using a commercially available NAD (from KOHJIN Life Sciences Co., Ltd.). Reaction conditions are listed below.
Reaction temperature: 30°C, 40°C, 50°C, 60°C, 70°C, 80°C
Reaction pH: pH1.0, pH2.0, pH3.0, pH4.0, pH5.0, pH6.0, pH7.0, pH8.0, pH9.0, pH10.0
Solvent composition:
   100 mM KCl-HCl (pH1.0)
   100 mM KCl-HCl (pH2.0)
   100 mM KHC₈H₄O₄-HCl (pH3.0)
   100 mM KHC₈H₄O₄-NaOH (pH4.0)
   100 mM MES-NaOH (pH5.0)
   100 mM MES-NaOH (pH6.0) 100 mM PIPES-NaOH (pH7.0)
   100 mM HEPES-NaOH (pH8.0)
   100 mM CHES-NaOH (pH9.0)
   100 mM CHES-NaOH (pH10.0)
Reaction time: 30 minutes
Amount of addition of enzyme: 1% (w/w) of enzyme composition prepared from Aspergillus niger was added relative to whole NAD.
Substrate concentration: 1% (w/v) NAD as final concentration

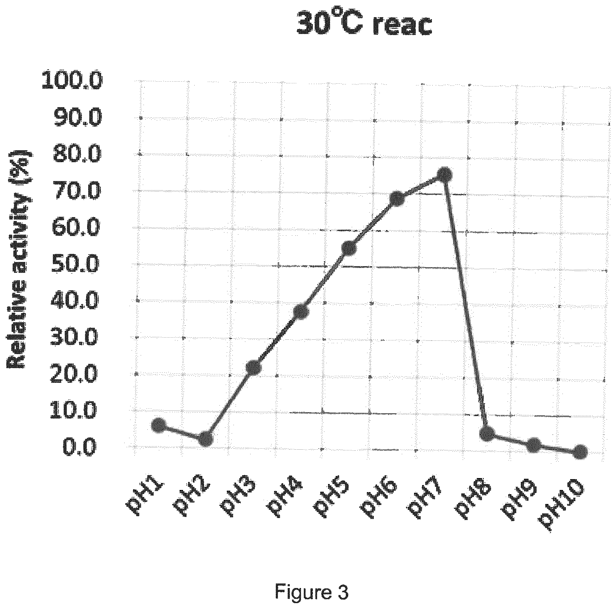

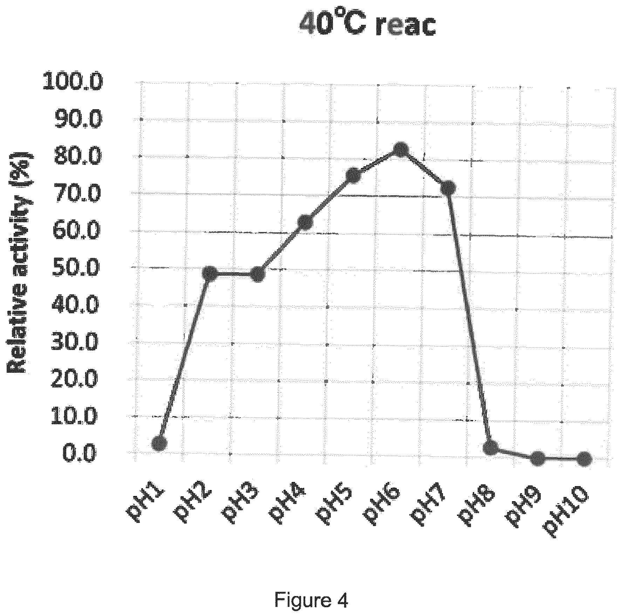

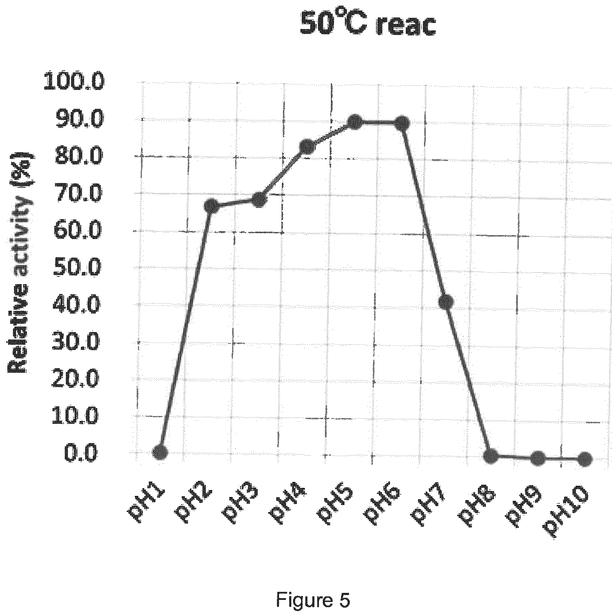

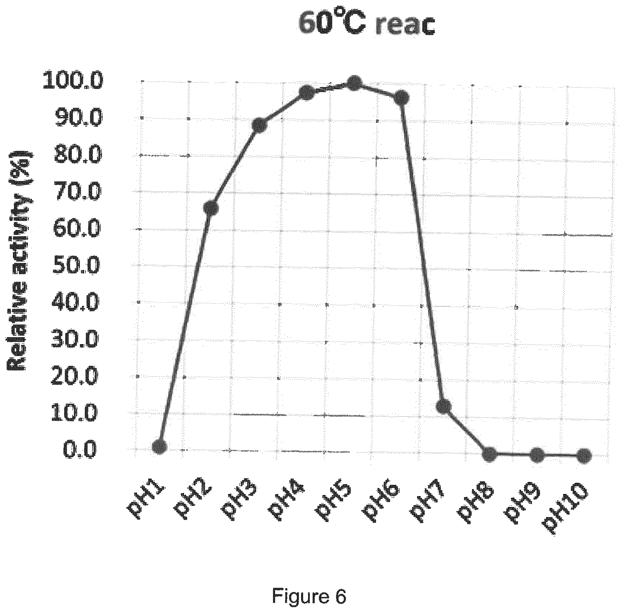

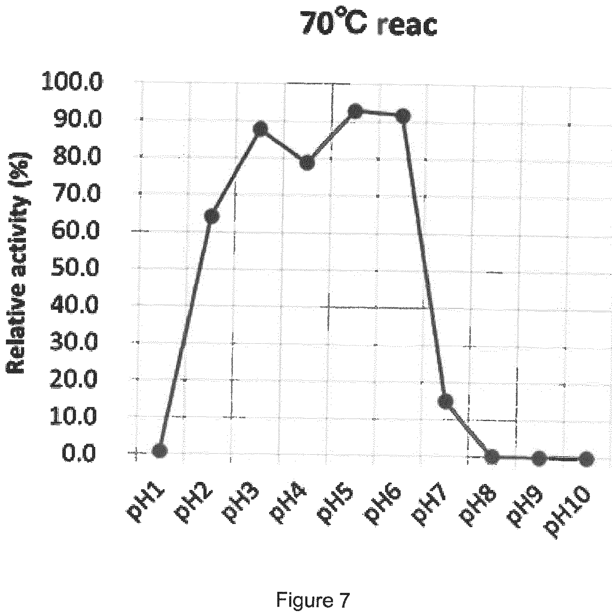

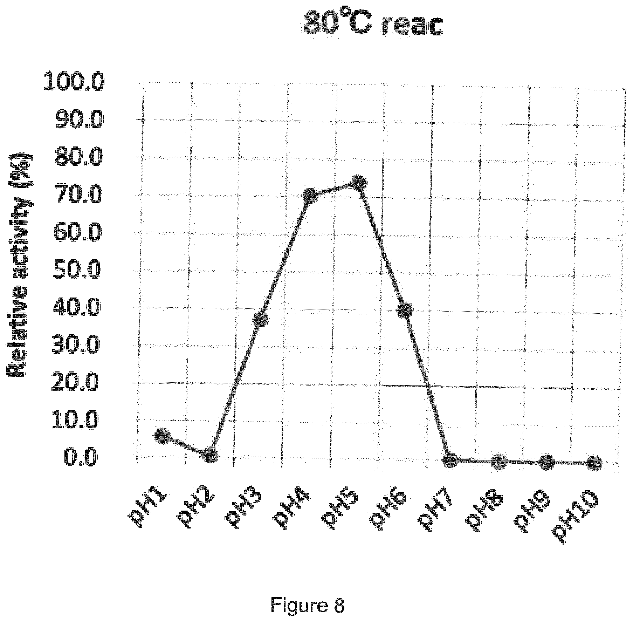

Results were illustrated in Figs. 3 to 8.

Note that β-NMN and a β-NMN-containing composition used for investigating optimum reaction conditions of the enzyme in the present application were measured under HPLC conditions described later in Examples. The individual β-NMN producing activities were given in terms of relative activities, while assuming β-NMN production after reaction at pH6.0 and 30°C as 100, which represents the maximum activity.

### <Culture of Yeast>

Candida utilis IAM 4264 was preliminarily seed-cultured in an Erlenmeyer flask that contains YPD medium (1% yeast extract, 2% polypeptone and 2% glucose), and the culture was then inoculated into 18 L medium kept in a 30-L fermenter, with the concentration adjusted to 1 to 2%. Chemical composition of the medium employed here was 4% glucose, 0.3% monoammonium phosphate, 0.161% ammonium sulfate, 0.137% potassium chloride, 0.08% magnesium sulfate, 1.6 ppm copper sulfate, 14 ppm iron sulfate, 16 ppm manganese sulfate, and 14 ppm zinc sulfate. Culture conditions employed were pH4.0, culture temperature of 30°C, aeration volume of 1 vvm, stirring speed of 600 rpm, wherein pH was controlled by adding ammonia. The cells were cultured for 16 hours, the culture fluid was collected, and the cells were collected by centrifugation, to thereby obtain 180 g of wet yeast cells.

The thus obtained yeast cells were washed by repetitively suspending them in distilled water and collecting them by centrifugation. The cells were re-suspended in distilled water so as to adjust the dry solid concentration to 82.88 g/L. The mixture was found to be pH5.8.

### <Preparation of Yeast Extract>

The cell suspension was kept on a water bath at 70°C, warmed up to 60°C under slow stirring, and then kept stirred for 10 minutes for extraction. After the extraction, 25 mL of the cell suspension was sampled and cooled on an ice bath, centrifuged at 10000 rpm for 10 minutes at 4°C, and the supernatant was collected. The precipitate was re-suspended in ultrapure water, whose volume of addition being equal to the supernatant, and the suspension was centrifuged to obtain the supernatant. The supernatant obtained by the first centrifugation and the supernatant obtained by the second centrifugation were pooled, and filled up by water to 50 mL, to thereby prepare an extraction liquid.

### <Advanced Fractionation of NAD from Yeast Extract>

The extraction liquid was allowed to pass through a cation exchange resin to condense NAD. The yeast extract after condensation was found to have an NAD content of 45% (w/w) .

### <Measurement of β-NMN Content under Optimum Enzyme Reaction Conditions>

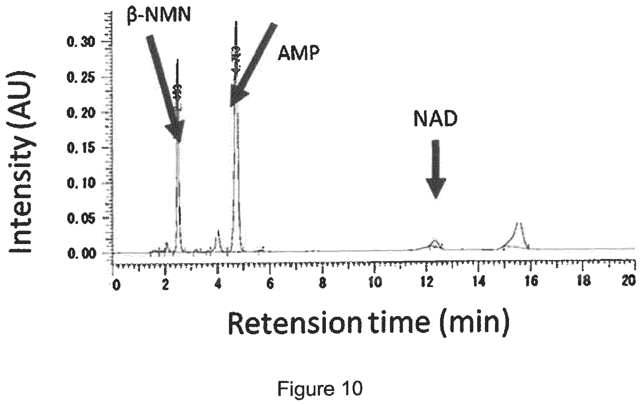

Yeast was cultured and extracted in the same way as described above by using Candida utilis IAM 4264, the yeast extraction liquid was conditioned to a temperature of 50°C and reaction pH to 6.0 by use of 9 N HCl or 9 N NaOH, to which a crude enzyme prepared from the aforementioned Aspergillus oryzae, or, a crude enzyme prepared from Aspergillus niger (ATCC10254) in the same way as from Aspergillus oryzae were added, while adjusting the amount of addition to 5% relative to the total content of NAD contained in the yeast extract, and an optimum reaction was allowed to proceed for 7 hours. After went through a drying process, a dry product of the yeast extract that contains β-NMN and a composition was obtained. The dry product was quantitatively analyzed according to the aforementioned measurement conditions. Chromatograms are shown in Figs. 10 and 11. β-NMN contained in the yeast extract obtained under the optimum enzyme reaction conditions was quantified to be 10% (w/w) (Aspergillus oryzae) and 20% (w/w) (Aspergillus niger), on the dry solid basis. The NAD content before reaction was 25% (w/w) on the dry solid basis, meanwhile the NAD content after reaction decreased down to 2% by weight on the dry solid basis, accompanied by production of β-NMN and AMP. From the finding, production of β-NMN by such enzyme reaction was presumed to follow the mechanism illustrated in Fig. 2.

Production of β-NMN was confirmed by using commercially available enzymes derived from the genus Aspergillus. NAD (from KOHJIN Life Sciences Co., Ltd.) was reacted with the commercially available enzymes enumerated below, under the conditions same as those in Examples of the present application, to confirm production of β-NMN. Also the yeast extraction liquid was prepared in the same way as in Example of the present application, to which any of commercially available enzymes including "Denazyme AP (genus Aspergillus) " (from Nagase ChemteX Corporation), "Deamizyme G (Aspergillus melleus)" (from Amano Enzyme Inc.), "Phytase Amano 3000 (Aspergillus niger)" (from Amano Enzyme Inc.), "Sumizyme LP50D (Aspergillus oryzae)" (from Shin Nihon Chemical Co., Ltd.), "Sumizyme PHY-G (Aspergillus niger)" from Shin Nihon Chemical Co., Ltd.), "Sumizyme PHYF -L (Aspergillus niger)" (from Shin Nihon Chemical Co., Ltd.) and "Sumizyme PHY (Aspergillus niger)" (from Shin Nihon Chemical Co., Ltd.) was added, with the amount of addition controlled to 1% (w/w) relative to the total content of NAD, and the optimum reaction was allowed to proceed for one hour. The reaction conditions involved pH6.0 and a temperature of 60°C. From the results, all enzymes were confirmed to produce β-NMN.

### Industrial Applicability

According to the present invention, a composition that contains β-NMN and AMP is obtainable from NAD, and also a yeast extract that contains β-NMN and AMP is obtainable from yeast having been proven by the history of safe use. The products of the present invention are ingestible not only as drug, but also as functional food or dietary supplement, and ingestion of the products of the present invention enables acquisition of functionality of β-NMN.

### Reference Signs List

1 NAD
2 β-NMN
3 AMP
4 A crude enzyme derived from a microorganism that belongs to the genus Aspergillus

## Claims

1. A method for producing β-nicotinamide mononucleotide, the method comprising performing a reaction of β-nicotinamide adenine dinucleotide which is a substrate, using a metabolic composition of a microorganism that belongs to genus Aspergillus.

2. A method for producing β-nicotinamide mononucleotide, the method comprising performing a reaction using an enzymatic protein derived from a microorganism that belongs to genus Aspergillus and having properties listed below:
(1) action: hydrolyzes a phosphoanhydride bond (pyrophosphate bond), which is a high-energy phosphate bond in a β-nicotinamide adenine dinucleotide structure, to produce β-nicotinamide mononucleotide and AMP;
(2) pyrophosphatase activity;
(3) optimum pH: pH3.0 to 7.0; and
(4) optimum temperature: 40°C to 70°C.

3. A method for producing a composition that contains 1.0% (w/w) or more of β-nicotinamide mononucleotide, the method comprising performing a reaction of β-nicotinamide adenine dinucleotide contained in an extract of yeast, or β-nicotinamide adenine dinucleotide which is a substrate, using an enzyme derived from a microorganism that belongs to genus Aspergillus and having properties listed below:
(1) action: hydrolyzes a phosphoanhydride bond (pyrophosphate bond), which is a high-energy phosphate bond in a β-nicotinamide adenine dinucleotide structure, to produce β-nicotinamide mononucleotide;
(2) pyrophosphatase activity;
(3) optimum pH: pH3.0 to 7.0; and
(4) optimum temperature: 40°C to 70°C.

4. An enzyme derived from a microorganism that belongs to genus Aspergillus, having physicochemical properties listed below:
(1) action: hydrolyzes a phosphoanhydride bond (pyrophosphate bond), which is a high-energy phosphate bond in a β-nicotinamide adenine dinucleotide structure, to produce β-nicotinamide mononucleotide;
(2) pyrophosphatase activity;
(3) optimum pH: pH3.0 to 7.0; and
(4) optimum temperature: 40°C to 70°C.
